# EUROPEAN PATENT APPLICATION

(11) **EP 4 640 826 A1**
(43) Date of publication of application: **29.10.2025**
(21) Application number: 24741754.6
(22) Date of filing: 12.01.2024
(51) Int. Cl.: C12N 9/00, C12N 15/77, C12P 21/02, C07K 14/34, C12R 1/15

(54) **NOVEL CARNOSINE SYNTHASE, AND METHOD FOR PRODUCING CARNOSINE BY USING SAME**

(30) Priority: 13.01.2023 KR 20230005632
(71) Applicant: CJ CheilJedang Corporation, Seoul 04560 (KR)
(72) Inventor: HWANG, Yeji, Seoul 04560 (KR); LEE, Heeseok, Seoul 04560 (KR); YOON, Byoung Hoon, Seoul 04560 (KR)
(74) Representative: Meissner Bolte Partnerschaft mbB
(86) International application number: PCT/KR2024/000627
(87) International publication number: WO 2024/151133

(57) **Abstract**

Provided are a polypeptide having carnosine synthase activity; a polynucleotide encoding the polypeptide; a microorganism including any one or more of the polypeptide, the polynucleotide, and a vector including the polynucleotide; a variant polypeptide having carnosine synthase activity; a polynucleotide encoding the variant polypeptide; a microorganism including any one or more of the variant polypeptide, the polynucleotide, and a vector including the polynucleotide; a composition for producing carnosine, the composition including any one or more of the polypeptide having carnosine synthase activity, the variant polypeptide, the microorganism, and a culture of the microorganism; a method for producing carnosine using any one or more of the polypeptide having carnosine synthase activity, the variant polypeptide, and the microorganism; and use of the polypeptide having carnosine synthase activity, the variant polypeptide, and the microorganism for producing carnosine.

## Description

### [Technical Field]

The present disclosure relates to a polypeptide having carnosine synthase activity; a polynucleotide encoding the polypeptide; a microorganism including any one or more of the polypeptide, the polynucleotide, and a vector including the polynucleotide; a variant polypeptide having carnosine synthase activity; a polynucleotide encoding the variant polypeptide; a microorganism including any one or more of the variant polypeptide, the polynucleotide, and a vector including the polynucleotide; a composition for producing carnosine, the composition including any one or more of the polypeptide having carnosine synthase activity, the variant polypeptide, the microorganism, and a culture of the microorganism; a method for producing carnosine using any one or more of the polypeptide having carnosine synthase activity, the variant polypeptide, and the microorganism; and use of the polypeptide having carnosine synthase activity, the variant polypeptide, and the microorganism for producing carnosine.

### [Background Art]

Carnosine is present in high concentrations in muscle and brain tissue, and is known to have antioxidant effects and muscle fatigue improvement effects as a physiologically active peptide.

Carnosine production can be achieved through enzymatic conversion (US 4359416 A). However, since it requires a large amount of substrate and ATP, there is a problem in that industrial mass production of carnosine is not easy in terms of cost.

### [Disclosure]

### [Technical Problem]

The problems to be solved in the present disclosure are to provide a polypeptide having carnosine synthase activity; a polynucleotide encoding the polypeptide; a microorganism including any one or more of the polypeptide, the polynucleotide, and a vector including the polynucleotide; a variant polypeptide having carnosine synthase activity; a polynucleotide encoding the variant polypeptide; a microorganism including any one or more of the variant polypeptide, the polynucleotide, and a vector including the polynucleotide; a composition for producing carnosine, the composition including any one or more of the polypeptide having carnosine synthase activity, the variant polypeptide, the microorganism, and a culture of the microorganism; a method for producing carnosine using any one or more of the polypeptide having carnosine synthase activity, the variant polypeptide, and the microorganism; and use of the polypeptide having carnosine synthase activity, the variant polypeptide, and the microorganism for producing carnosine.

### [Technical Solution]

An object of the present disclosure is to provide a polypeptide having carnosine synthase activity, the polypeptide including an amino acid sequence of SEQ ID NO: 2.

Another object of the present disclosure is to provide a polynucleotide encoding the polypeptide having carnosine synthase activity.

Still another object of the present disclosure is to provide a microorganism including any one or more of the polypeptide having carnosine synthase activity, the polynucleotide encoding the same, and a vector including the polynucleotide.

Still another object of the present disclosure is to provide a variant polypeptide having carnosine synthase activity, in which an amino acid corresponding to position 108 and an amino acid corresponding to position 378 from the N-terminus of SEQ ID NO: 2 are substituted with different amino acids.

Still another object of the present disclosure is to provide a polynucleotide encoding the variant polypeptide.

Still another object of the present disclosure is to provide a microorganism including any one or more of the variant polypeptide, the polynucleotide encoding the same, and a vector including the polynucleotide.

Still another object of the present disclosure is to provide a composition for producing carnosine, the composition including any one or more of the polypeptide having carnosine synthase activity; the microorganism including any one or more of the polypeptide having carnosine synthase activity, the polynucleotide encoding the same, and a vector including the polynucleotide; a culture of the microorganism; the variant polypeptide; the microorganism including any one or more of the variant polypeptide, the polynucleotide encoding the same, and a vector including the polynucleotide; and a culture of the microorganism.

Still another object of the present disclosure is to provide a method for producing carnosine, the method including the step of culturing the microorganism.

Still another object of the present disclosure is to provide use of the polypeptide having carnosine synthase activity, the variant polypeptide, and the microorganism for producing carnosine.

### [Advantageous Effects]

Efficient carnosine production is possible using a polypeptide having carnosine synthase activity of the present disclosure, a variant polypeptide, a polynucleotide, a microorganism, a composition, and/or a method.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a polypeptide having carnosine synthase activity, the polypeptide including an amino acid sequence of SEQ ID NO: 2.

As used herein, the term "carnosine synthase" refers to an enzyme capable of catalyzing a reaction that produces carnosine using beta alanine and L-histidine as substrates, and belongs to the ligase family, and thus may be used interchangeably with L-ligase and LAL. A specific example of carnosine synthase may include an enzyme including the amino acid sequence of SEQ ID NO: 2, but is not limited thereto.

As used herein, the term "polypeptide having carnosine synthase activity" refers to a polypeptide having carnosine synthase activity, the polypeptide including the amino acid sequence of SEQ ID NO: 2.

In one embodiment, the polypeptide having carnosine synthase activity in the present disclosure may include the amino acid sequence of SEQ ID NO: 2 or an amino acid sequence having 60% or more homology or identity thereto, but is not limited thereto as long as it has carnosine synthase activity. The amino acid sequence may be a polypeptide including the amino acid sequence of SEQ ID NO: 2 or an amino acid sequence having at least 60%, 70%, 80%, 85%, 90%, 95%, 96%, 97%, 98%, or 99% or more homology or identity thereto, and may have or include an amino acid sequence having the above homology or identity, or may consist of the amino acid sequence, or may essentially consist of the amino acid sequence. It is not known whether or not SEQ ID NO: 2 has carnosine synthase activity, but the amino acid sequence of SEQ ID NO: 2 itself may be obtained from NCBI's GenBank or Kyoto Encyclopedia of Genes and Genomes (KEGG), which is a known database. For example, the polypeptide having carnosine synthase activity may be derived from the genus *Streptococcus* or *Streptococcus pneumoniae,* but is not limited thereto.

Further, in the present disclosure, although the protein including the amino acid sequence of SEQ ID NO: 2 was defined as an example of carnosine synthase, it is also apparent that a polypeptide having an amino acid sequence having deletion, modification, substitution, or addition of some sequence also falls within the scope of the present disclosure as long as the amino acid sequence has such a homology or identity and exhibits activity identical or corresponding to that of the protein consisting of the amino acid sequence of SEQ ID NO: 2. Examples thereof do not exclude addition of a sequence, which does not alter the function of the protein, upstream or downstream of the amino acid sequence of SEQ ID NO: 2, a mutation that may occur naturally, a silent mutation thereof, or a conservative substitution, and it would be obvious that even a protein with such a sequence addition or mutation falls within the scope of the present disclosure, as long as it has activity identical or corresponding to that of the protein.

Further, the carnosine synthase protein having the amino acid sequence of SEQ ID NO: 2 may be encoded by a polynucleotide having or including a sequence of SEQ ID NO: 11 or a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ **ID** NO: 11, or a polynucleotide consisting of or essentially consisting of the sequence of SEQ ID NO: 11 or a nucleotide sequence having 60% or more, 70% or more, 75% or more, 80% or more, 85% or more, 90% or more, 95% or more, 96% or more, 97% or more, 98% or more, and less than 100% homology or identity to the sequence of SEQ **ID** NO: 11, but is not limited thereto.

Another aspect of the present disclosure provides a variant polypeptide having carnosine synthase activity, in which any one or more of an amino acid corresponding to position 108 and an amino acid corresponding to position 378 from the N-terminus of SEQ ID NO: 2 are substituted with different amino acids.

As used herein, the term "variant polypeptide having carnosine synthase activity" refers to a variant polypeptide having carnosine synthase activity, in which any one or more of the amino acid corresponding to position 108 and the amino acid corresponding to position 378 from the N-terminus of SEQ ID NO: 2 are substituted with different amino acids. In the present disclosure, the "variant polypeptide having carnosine synthase activity" may be used interchangeably with a "variant polypeptide".

In one embodiment, the reference carnosine synthase subject to mutation in the present disclosure is the same as the above-described polypeptide having carnosine synthase activity.

As used herein, the term "variant" refers to a polypeptide or protein, in which one or more amino acids differ from those of the recited sequence in conservative substitution and/or modification, but the functions or properties of the polypeptide or protein are maintained.

Such a variant may generally be identified by modifying one or more amino acids of the amino acid sequence of the polypeptide or protein and evaluating the properties of the modified polypeptide or protein. In other words, the ability of the variant may be increased, unchanged, or decreased as compared to that of the protein before being varied. Further, some variants may include variants in which one or more portions such as an N-terminal leader sequence or a transmembrane domain have been removed. Other variants may include variants in which a portion of the N- and/or C-terminus has been removed from the mature protein. The term "variant" may be used interchangeably with terms such as modification, modified polypeptide, modified protein, mutant, mutein, and divergent and is not limited thereto as long as it is a term used with the meaning of variation.

As used herein, the "conservative substitution" means substitution of one amino acid with another amino acid having similar structural and/or chemical properties. Usually, conservative substitution may hardly affect or not affect the activity of produced proteins or polypeptides. Further, the variant may have one or more conservative substitutions while still retaining one or more biological activities.

Such amino acid substitution may generally occur based on similarity of polarity, charge, solubility, hydrophobicity, hydrophilicity, and/or amphipathic nature of a residue. For example, positively charged (basic) amino acids include arginine, lysine, and histidine; negatively charged (acidic) amino acids include glutamic acid and aspartate; aromatic amino acids include phenylalanine, tryptophan and tyrosine; and hydrophobic amino acids include alanine, valine, isoleucine, leucine, methionine, phenylalanine, tyrosine, and tryptophan. Further, amino acids may be classified into amino acids with electrically charged side chains and amino acids with uncharged side chains. The amino acids with electrically charged side chains include aspartic acid, glutamic acid, lysine, arginine, and histidine. The amino acids with uncharged side chains may be further classified into non-polar amino acids or polar amino acids, wherein the non-polar amino acids include glycine, alanine, valine, leucine, isoleucine, methionine, phenylalanine, tryptophan, and proline, and the polar amino acids include serine, threonine, cysteine, tyrosine, asparagine, and glutamine, but are not limited thereto.

Further, the polypeptide having carnosine synthase activity and the variant polypeptide having carnosine synthase activity may include deletions or additions of amino acids that have minimal effect on the properties and secondary structure of the polypeptide. For example, the polypeptide may be conjugated with a protein N-terminal signal (or leader) sequence that is co-translationally or post-translationally involved in the protein transfer. Further, the polypeptide may be conjugated with other sequences or linkers so as to identify, purify, or synthesize the polypeptide.

In one embodiment, the variant polypeptide of the present disclosure may be a variant polypeptide having carnosine synthase activity, in which the amino acid corresponding to position 108 and the amino acid corresponding to position 378 from the N-terminus of SEQ ID NO: 2 are substituted with different amino acids.

As used herein, the 'substitution with different amino acids' is not limited as long as the substituted amino acid differs from the amino acid before substitution. Meanwhile, in the present disclosure, when expressed as 'a specific amino acid is substituted', it is obvious that the amino acid is substituted with an amino acid different from the amino acid before substitution, even though it is not separately indicated that the amino acid is substituted with a different amino acid.

The "position N" of the present disclosure may include the position N and an amino acid position corresponding to the position N. For example, the position N may include an amino acid position corresponding to an arbitrary amino acid residue in a mature polypeptide, disclosed in a specific amino acid sequence. For example, the specific amino acid sequence may be the amino acid sequence of SEQ ID NO: 2.

As used herein, the term "corresponding to" refers to an amino acid residue at a position listed in a polypeptide, or an amino acid residue similar to, identical to, or homologous to a residue listed in a polypeptide. Identifying the amino acid at the corresponding position may be determining a specific amino acid in a sequence that refers to a specific sequence. As used herein, the "corresponding region" generally refers to a similar or corresponding position in a related protein or a reference protein.

For example, an arbitrary amino acid sequence is aligned with SEQ ID NO: 2, and based on this, each amino acid residue of the amino acid sequence may be numbered with reference to the numerical position of the amino acid residue corresponding to the amino acid residue of SEQ ID NO: 2. For example, a sequence alignment algorithm as described in the present disclosure may determine the position of an amino acid, or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000), Trends Genet. 16: 276-277), etc. may be used, but are not limited thereto, and a sequence alignment program, a pairwise sequence comparison algorithm, etc., which known in the art, may be appropriately used.

In one embodiment, the variant polypeptide of the present disclosure may have substitutions of the amino acid corresponding to position 108 and the amino acid corresponding to position 378 from the N-terminus of SEQ ID NO: 2 with different amino acids while having 60% or more and less than 100% sequence homology, specifically, 80% or more and less than 100% sequence homology to the amino acid sequence of SEQ ID NO: 2, but is not limited thereto.

In one embodiment of the above-described embodiments, the variant polypeptide of the present disclosure may include substitutions of the amino acid corresponding to position 108 and the amino acid corresponding to position 378 from the N-terminus of SEQ ID NO: 2 with different amino acids in an amino acid sequence having at least 60%, 70%, 75%, 80%, 85%, 90%, 95%, 96%, 97%, 98%, 99%, 99.5%, 99.7%, 99.8%, or 99.9% or more homology or identity to the amino acid sequence of SEQ ID NO: 2. It is also apparent that a variant having an amino acid sequence having deletion, modification, substitution, conservative substitution, or addition of some sequence also falls within the scope of the present disclosure as long as the amino acid sequence has such a homology or identity and exhibits activity corresponding to that of the variant polypeptide of the present disclosure.

In one embodiment, with regard to the variant polypeptide of the present disclosure, the amino acid corresponding to position 108 and the amino acid corresponding to position 378 from the N-terminus of SEQ ID NO: 2 are substituted with glutamic acid and lysine, respectively.

In one embodiment, with regard to the variant polypeptide of the present disclosure, asparagine which is the amino acid corresponding to position 108 and histidine which is the amino acid corresponding to position 378 from the N-terminus of SEQ ID NO: 2 are substituted with glutamic acid and lysine, respectively.

In one embodiment of the above-described embodiments, the variant polypeptide of the present disclosure may include an amino acid sequence having at least 60%, 70%, 75%, 80%, 85%, 90%, 95%, 96%, 97%, 98%, 99%, 99.5%, 99.7%, 99.8%, or 99.9% or more homology or iden tity to SEQ ID NO: 2 while fixing the amino acid corresponding to position 108 and the amino acid corresponding to position 378 in the amino acid sequence of SEQ ID NO: 2 by substituting with glutamic acid and lysine, respectively.

In one embodiment, the variant polypeptide of the present disclosure may include an amino acid sequence of SEQ ID NO: 8. The variant polypeptide of the present disclosure may have or include an amino acid sequence having at least 60%, 70%, 80%, 85%, 90%, 95%, 96%, 97%, 98%, or 99% or more homology or identity to SEQ ID NO: 8, or may consist of the amino acid sequence, or may essentially consist of the amino acid sequence.

Further, with regard to the variant polypeptide of the present disclosure, it is also apparent that a protein having an amino acid sequence having deletion, modification, substitution, conservative substitution, or addition of some sequence, in addition to the amino acids at positions 108 and 378, also falls within the scope of the present disclosure as long as the amino acid sequence has activity identical or corresponding to that of the protein consisting of the amino acid sequence of SEQ ID NO: 8. Examples thereof do not exclude addition of a sequence, which does not alter the function of the protein, upstream or downstream of the amino acid sequence, a mutation that may occur naturally, a silent mutation thereof, or a conservative substitution, as long as it has activity identical or corresponding to that of the variant polypeptide, and it would be obvious that even a protein with such a sequence addition or mutation falls within the scope of the present disclosure.

In one embodiment, the variant polypeptide of the present disclosure may have enhanced carnosine synthase activity, as compared to the polypeptide before modification, but is not limited thereto.

Still another aspect of the present disclosure provides a polynucleotide encoding the polypeptide having carnosine synthase activity of the present disclosure.

Still another aspect of the present disclosure provides a polynucleotide encoding the variant polypeptide of the present disclosure.

As used herein, the term "polynucleotide" is a DNA or RNA strand having a certain length or more as a polymer of nucleotides in which nucleotide monomers are connected in a long chain by covalent bonds, and more specifically, it refers to a polynucleotide fragment encoding the polypeptide having the carnosine synthase activity and/or the variant polypeptide.

The polynucleotide encoding the polypeptide having carnosine synthase activity of the present disclosure and/or the variant polypeptide may include any polynucleotide sequence without limitation as long as it encodes a polypeptide having activity corresponding to that of the polypeptide having carnosine synthase activity of the present disclosure and/or the variant polypeptide.

The polynucleotide encoding the polypeptide having carnosine synthase activity of the present disclosure and/or the variant polypeptide may undergo various modifications in the coding region within the scope that does not change the amino acid sequence of the polypeptide having carnosine synthase activity of the present disclosure and/or the variant polypeptide in consideration of codon degeneracy or codons preferred in organisms that are intended to express the polypeptide having carnosine synthase activity of the present disclosure and/or the variant polypeptide. Therefore, it is apparent that a variant having a polynucleotide sequence having deletion, modification, substitution, conservative substitution, or addition of some sequence also falls within the scope of the present disclosure as long as the polynucleotide sequence is able to encode the amino acid sequence of the polypeptide having carnosine synthase activity of the present disclosure and/or the variant polypeptide or the polypeptide having homology or identity thereto, in consideration of codon degeneracy or codons preferred in organisms that are intended to express the polypeptide having carnosine synthase activity of the present disclosure and/or the variant polypeptide.

In one embodiment, the polynucleotide encoding the polypeptide having carnosine synthase activity of the present disclosure may have or include a nucleotide sequence having 60%, 70%, 75%, 80%, 85%, 90%, 95%, 96%, 97%, 98%, 99%, 99.5%, 99.7%, or 99.9% or more homology or identity to a sequence of SEQ ID NO: 11, or may consist of or essentially consist of the nucleotide sequence, but is not limited thereto.

For example, the polynucleotide encoding the polypeptide having carnosine synthase activity of the present disclosure may be SEQ ID NO: 11 or a degenerated sequence thereof.

In one embodiment, the polynucleotide encoding the variant polypeptide of the present disclosure may include substitutions of a codon encoding the amino acid corresponding to position 108 and a codon encoding the amino acid corresponding to position 378 from the N-terminus of SEQ ID NO: 2 with codons encoding different amino acids (e.g., glutamic acid or lysine) in a nucleotide sequence having 60%, 70%, 75%, 80%, 85%, 90%, 95%, 96%, 97%, 98%, 99%, 99.5%, 99.7%, or 99.9% or more homology or identity to the sequence of SEQ ID NO: 11, but is not limited thereto.

For example, with regard to the polynucleotide encoding the variant polypeptide of the present disclosure, the codon encoding the amino acid corresponding to position 108 and/or the codon encoding the amino acid corresponding to position 378 from the N-terminus of SEQ ID NO: 2 may be a codon encoding glutamic acid and/or a codon encoding lysine, respectively in a nucleotide sequence having 60%, 70%, 75%, 80%, 85%, 90%, 95%, 96%, 97%, 98%, 99%, 99.5%, 99.7%, or 99.9% or more homology or identity to the sequence of SEQ ID NO: 11.

For another example, the polynucleotide encoding the variant polypeptide of the present disclosure may be SEQ ID NO: 17 or a degenerated sequence thereof.

In one embodiment, the polynucleotide encoding the variant polypeptide of the present disclosure may have or include SEQ ID NO: 17, or a nucleotide sequence having 60%, 70%, 75%, 80%, 85%, 90%, 95%, 96%, 97%, 98%, 99%, 99.5%, 99.7%, or 99.9% or more, and less than 100% homology or identity thereto, or may consist of or essentially consist of the nucleotide sequence, but is not limited thereto.

Further, the polynucleotide encoding the polypeptide having carnosine synthase activity of the present disclosure and the polynucleotide encoding the variant polypeptide of the present disclosure may include a probe that may be prepared from a known gene sequence, for example, may include any sequence without limitation as long as it is a sequence that is able to hybridize with a complementary sequence to the entirety or a part of the polynucleotide sequence of the present disclosure under stringent conditions.

The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having higher homology or identity, i.e., polynucleotides having 60% or more, 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1XSSC, 0.1% SDS, specifically, 60°C, 0.1XSSC, 0.1% SDS, more specifically 68°C, 0.1XSSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also include substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

As used herein, the term "homology" or "identity" means relevance between two given amino acid sequences or base sequences and may be expressed as a percentage. The terms "homology and identity" may often be used interchangeably.

The sequence homology or identity of a conserved polynucleotide or polypeptide is determined by standard alignment algorithms, and the default gap penalty established by a program used may be used together. Substantially, homologous or identical sequences are generally capable of being hybridized with the entirety or a part of the sequence under moderately or highly stringent conditions. It is apparent that hybridization also includes hybridization of a polynucleotide with a polynucleotide including a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package ((Devereux, J., et al, Nucleic Acids Research 12: 387(1984)), BLASTP, BLASTN, FASTA(Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST of the National Center for Biotechnology Information or ClustalW may be used to determine the homology, similarity, or identity.

The homology, similarity, or identity between polynucleotides or polypeptides may be determined by comparing sequence information using the GAP computer program as introduced by, for example, Needleman et al.(1970), J Mol Biol. 48:443, as disclosed by Smith and Waterman, Adv. Appl. Math(1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may include: (1) a binary comparison matrix (containing a value 1 for identity and a value 0 for non-identity) and the weighted comparison matrix of Gribskov et al(1986) Nucl. Acids Res. 14: 6745 as disclosed in Schwartz and Dayhoff, eds., Atlas Of Protein Sequence And Structure, National Biomedical Research Foundation, pp. 353-358(1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps. Therefore, the term "homology" or "identity" used herein represents the relevance between sequences.

Still another aspect of the present disclosure provides a vector including the polynucleotide encoding the polypeptide having carnosine synthase activity of the present disclosure and/or the variant polypeptide.

The polynucleotide is as described in other aspects.

The vector may be an expression vector for expressing the polynucleotide in a microorganism, but is not limited thereto.

As used herein, the term "vector" may include a DNA construct including a nucleotide sequence of a polynucleotide encoding a polypeptide of interest which is operably linked to a suitable expression regulatory region (or expression control sequence) so that the polypeptide of interest may be expressed in a suitable host. The expression regulatory region may include a promoter capable of initiating transcription, any operator sequence for controlling the transcription, a sequence encoding a suitable mRNA ribosome binding site, and a sequence controlling termination of transcription and translation. The vector may be transformed into a suitable microorganism, and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pET system, etc. may be used as a plasmid vector. Specifically, pDZ (Korean Patent Publication No. 10-0924065 and International Publication Patent No. 2008-033001), pDZTn (Korean Patent No. 10-1126041), pDC, pDCM2, pACYC177, pACYC184, pCL, pECCG117, pUC19, pBR322, pMW118, pCC1BAC vector, etc. may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further include a selection marker for the confirmation of chromosome insertion. The selection marker is for selecting the cells transformed with vectors, i.e., for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector including a polynucleotide encoding a target polypeptide is introduced into a microorganism or a microorganism so that the polypeptide encoded by the polynucleotide may be expressed in the microorganism. The transformed polynucleotide may be located regardless of the position, either by being inserted into the chromosome of the microorganism or located outside the chromosome as long as it may be expressed in the microorganism. Further, the polynucleotide includes DNA and/or RNA encoding a polypeptide of interest. The polynucleotide may be introduced in any form as long as it may be introduced into a microorganism and expressed. For example, the polynucleotide may be introduced into a microorganism in the form of an expression cassette, which is a gene construct containing all elements required for self-expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a microorganism in its own form and operably linked to a sequence required for expression in the microorganism, but is not limited thereto.

The method of transforming the vector of the present disclosure includes any method of introducing a nucleic acid into a cell, and may be performed by selecting an appropriate standard technique, as known in the art, according to the host cell. For example, the method may include electroporation, calcium phosphate (CaPO₄) precipitation, calcium chloride (CaCl₂) precipitation, microinjection, a polyethylene glycol (PEG) method, a DEAE-dextran method, a cationic liposome method, and a lithium acetate-DMSO method, etc., but is not limited thereto.

Further, the term "operably linked" means that the polynucleotide sequence is functionally linked to a promoter sequence that initiates and mediates transcription of the polynucleotide encoding the polypeptide having carnosine synthase activity of the present disclosure and/or the variant polypeptide.

Still another aspect of the present disclosure provides a microorganism including any one or more of the polypeptide having carnosine synthase activity of the present disclosure, the polynucleotide encoding the same, and the vector including the polynucleotide.

Still another aspect of the present disclosure provides a microorganism including any one or more of the variant polypeptide of the present disclosure, the polynucleotide encoding the same, and the vector including the polynucleotide.

The polypeptide having carnosine synthase activity, the variant polypeptide, the polynucleotide, and the vector are as described in other aspects.

As used herein, the term "microorganism (or strain)" includes all of wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism including genetic modification for production of carnosine. As used herein, the "strain" and "microorganism" have the same meaning and may be used interchangeably without limitation.

The microorganism of the present disclosure may be a microorganism in which *panD* gene encoding aspartic acid 1-decarboxylase protein required to convert aspartic acid to beta-alanine is introduced into the microorganism to exhibit the protein activity or to have the enhanced activity such that a microorganism inherently having no carnosine-producing ability has the carnosine-producing ability or a microorganism having the carnosine-producing ability has the further enhanced carnosine-producing ability. The aspartic acid 1-decarboxylase protein is not limited as long as it is a protein exhibiting the activity identical or similar thereto. For a specific example, the aspartic acid 1-decarboxylase protein may consist of or include an amino acid sequence of SEQ ID NO: 9, but may consist of or include an amino acid sequence having the activity corresponding to the aspartic acid 1-decarboxylase while having at least 60%, 65%, 70%, 75%, 80%, 85%, 86%, 87%, 88%, 89%, 90%, 91%, 92%, 93%, 94%, 95%, 96%, 97%, 98%, or 99% homology or identity to the amino acid sequence. It is also apparent that a protein having deletion, modification, substitution, or addition of some sequence is included in the aspartic acid 1-decarboxylase as long as the protein has the homology or identity while exhibiting activity corresponding to that of the aspartic acid 1-decarboxylase. Further, for a specific example, the polynucleotide encoding the aspartic acid 1-decarboxylase protein may consist of or include a sequence of SEQ ID NO: 18. The polynucleotide undergoes various modifications in the coding region within the scope that does not change the amino acid sequence in consideration of codon degeneracy or codons preferred in the microorganism of the present disclosure. Specifically, the polynucleotide may consist of or include the nucleotide sequence of SEQ ID NO: 18 or a nucleotide sequence having 60%or more, 70% or more, 80% or more, 85% or more, 90% or more, 95% or more, 96% or more, 97% or more, 98% or more, and less than 100% homology or identity to the sequence of SEQ ID NO: 18, but is not limited thereto.

In one embodiment, the microorganism of the present disclosure may be a microorganism of the genus *Enterobacter,* the genus *Escherichia,* the genus *Erwinia,* the genus *Serratia,* the genus *Providencia,* the genus *Corynebacterium,* or the genus *Brevibacterium.* More specifically, it may be a microorganism of the genus *Corynebacterium,* but is not limited thereto.

In one embodiment of the above-described embodiments, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris*, or *Corynebacterium flavescens.* Specifically, it may be *Corynebacterium glutamicum,* but is not limited thereto.

In one embodiment of the microorganism of the present disclosure, the microorganism including any one or more of the polypeptide having carnosine synthase activity of the present disclosure, the polynucleotide encoding the same, and the vector including the polynucleotide may have the further enhanced activity of AroP (Aromatic amino acid transport protein) protein, but is not limited thereto.

In one embodiment of the above-described embodiments, in the microorganism including any one or more of the polypeptide having carnosine synthase activity of the present disclosure, the polynucleotide encoding the same, and the vector including the polynucleotide, the carnosine-producing ability may be increased by further enhancing the activity of AroP protein.

In one embodiment of the microorganism of the present disclosure, the microorganism including any one or more of the variant polypeptide of the present disclosure, the polynucleotide encoding the same, and the vector including the polynucleotide may have the further enhanced activity of AroP protein, but is not limited thereto.

In one embodiment of the above-described embodiments, in the microorganism including any one or more of the variant polypeptide of the present disclosure; the polynucleotide encoding the same; and the vector including the polynucleotide, the carnosine-producing ability may be increased by further enhancing the activity of AroP protein.

As used herein, the term "AroP protein" refers to an aromatic amino acid transport protein, and may be used interchangeably with "AroP". The aromatic amino acid may include phenylalanine, tryptophan, and tyrosine. Further, the AroP may be encoded by *aroP* gene, but is not limited thereto.

In one embodiment, the AroP protein of the present disclosure may include an amino acid sequence of SEQ ID NO: 19 or an amino acid sequence having 60% or more homology or identity thereto, but is not limited thereto. The amino acid sequence may be a polypeptide including SEQ ID NO: 19 or an amino acid sequence having at least 60%, 70%, 80%, 85%, 90%, 95%, 96%, 97%, 98%, or 99% or more homology or identity thereto. The sequence of SEQ ID NO: 19 may be obtained from NCBI's GenBank or Kyoto Encyclopedia of Genes and Genomes (KEGG), which is a known database. For example, the AroP protein subject to enhancement may be derived from the genus *Corynebacterium* or *Corynebacterium glutamicum,* but is not limited thereto.

Further, in the present disclosure, although the protein including the amino acid sequence of SEQ ID NO: 19 was defined as an example of AroP protein, it is also apparent that a polypeptide having an amino acid sequence having deletion, modification, substitution, or addition of some sequence also falls within the scope of the AroP protein of the present disclosure as long as the amino acid sequence has such a homology or identity and exhibits activity identical or corresponding to that of the protein consisting of the amino acid sequence of SEQ ID NO: 19. Examples thereof do not exclude addition of a sequence, which does not alter the function of the protein, upstream or downstream of the amino acid sequence of SEQ ID NO: 19, a mutation that may occur naturally, a silent mutation thereof, or a conservative substitution, and it would be obvious that even a protein with such a sequence addition or mutation falls within the scope of the present disclosure, as long as it has activity identical or corresponding to that of the protein.

Further, the AroP protein having the amino acid sequence of SEQ ID NO: 19 may be encoded by a polynucleotide having or including a sequence of SEQ ID NO: 20 or a nucleotide sequence having 60% or more, 70% or more, 75% or more, 80% or more, 85% or more, 90% or more, 95% or more, 96% or more, 97% or more, 98% or more, and less than 100% homology or identity thereto, or consisting of or essentially consisting of the nucleotide sequence, but is not limited thereto.

As used herein, the term "enhancement" of polypeptide or protein activity means that the activity of a polypeptide or protein is increased, as compared to the endogenous activity. The enhancement may be used interchangeably with terms such as activation, up-regulation, overexpression, and increase, etc. The activation, enhancement, up-regulation, overexpression, and increase may include both exhibiting activity that was not originally possessed and exhibiting improved activity as compared to the endogenous activity or activity before modification.

The "endogenous activity" means the activity of a specific polypeptide or protein originally possessed by a parent strain before the trait is changed or an unmodified microorganism, when the trait is changed by genetic variation due to natural or artificial factors. This may be used interchangeably with "activity before modification". The fact that the activity of a polypeptide or protein is "enhanced", "up-regulated", "overexpressed", or "increased" as compared to the endogenous activity means that the activity of the polypeptide is improved as compared to the activity and/or concentration (expression level) of a specific polypeptide or protein originally possessed by a parent strain before the trait is changed or an unmodified microorganism.

The enhancement may be achieved through the introduction of a foreign polypeptide or protein or the enhancement of activity and/or concentration (expression level) of the foreign and/or endogenous polypeptide or protein. The enhancement of the activity of the polypeptide or protein may be confirmed by an increase in the degree of activity and the expression level of the corresponding polypeptide or protein or in the amount of the product produced from the corresponding protein.

For the enhancement of the activity of the polypeptide or protein, various methods well known in the art may be applied, and the method is not limited as long as the activity of the polypeptide or protein of interest may be enhanced as compared to that of the microorganism before being modified. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the activity enhancement of the polypeptide or protein of the present disclosure may be:
1) increase in the intracellular copy number of the polynucleotide encoding the polypeptide or protein;
2) replacement of a gene expression regulatory region on a chromosome encoding the polypeptide or protein with a sequence exhibiting strong activity;
3) modification of a nucleotide sequence encoding an initiation codon or **5'-UTR** region of the gene transcript encoding the polypeptide or protein;
4) modification of the amino acid sequence of the polypeptide or protein to enhance the activity of the polypeptide or protein;
5) modification of the polynucleotide sequence encoding the protein to enhance the activity of the polypeptide or protein (e.g., modification of the polynucleotide sequence of the protein gene to encode the protein that has been modified to enhance the activity of the protein);
6) introduction of a foreign protein exhibiting the activity of the polypeptide or protein or a foreign polynucleotide encoding the same;
7) codon optimization of the polynucleotide encoding the polypeptide or protein;
8) analysis of the tertiary structure of the polypeptide or protein to select and to modify or chemically modify the exposed site; or
9) a combination of two or more selected from 1) to 8), but is not particularly limited thereto.

In the above, the increase in the copy number of the gene may be performed, but is not particularly limited to, in the form of being operably linked to a vector or by inserting into the chromosome of a host cell. Specifically, a vector, which is operably linked to the polynucleotide encoding the protein of the present disclosure and is able to replicate and to function irrespective of a host cell, may be introduced into the host cell. Alternatively, a vector, which is operably linked to the polynucleotide and is able to insert the polynucleotide into the chromosome of a host cell, may be introduced into the chromosome of the host cell. The insertion of the polynucleotide into the chromosome may be performed by any method known in the art, e.g., homologous recombination.

Next, the modification of an expression regulatory sequence to increase expression of the polynucleotide may be performed by, but is not particularly limited to, inducing a variation in a nucleotide sequence due to deletion, insertion, nonconservative or conservative substitution, or a combination thereof, or replacing with a nucleotide sequence having stronger activity so that the activity of the expression regulatory sequence is further enhanced. The expression regulatory sequence may include, but is not particularly limited to, a promoter, an operator sequence, a sequence encoding a ribosome binding site, a sequence controlling the termination of transcription and translation, etc.

A strong promoter instead of the original promoter may be linked to the upstream of the polynucleotide expression unit, but is not limited to thereto. Examples of known strong promoters include CJ1 to CJ7 promoters (used interchangeably with cj1 to cj7, respectively; US Patent No. US 7662943 B2), pyk promoter, lac promoter, trp promoter, trc promoter, tac promoter, lambda phage PR promoter, PL promoter, tet promoter, gapA promoter, SPL7 promoter, SPL13(sm3) promoter (US Patent No. US 10584338 B2), O2 promoter (US Patent No. US 10273491 B2), tkt promoter, yccA promoter, etc., but is not limited thereto.

Furthermore, the modification of the polynucleotide sequence on the chromosome may be performed by, but is not particularly limited to, inducing a variation on the expression regulatory sequence due to deletion, insertion, nonconservative or conservative substitution, or a combination thereof, or replacing with a polynucleotide sequence that is improved to have stronger activity so that the activity of the polynucleotide sequence is further enhanced.

In one embodiment, with regard to the variant polypeptide having carnosine synthase activity of the present disclosure, the amino acid sequence of an unmodified polypeptide (e.g., SEQ ID NO: 2) having carnosine synthase activity is modified in order to enhance activity thereof, thereby enhancing the carnosine synthase activity of the variant polypeptide. In the present disclosure, the polypeptide having carnosine synthase activity and the variant polypeptide may be further enhanced (e.g., replacement of the sequence of the gene expression regulatory region thereof).

In another embodiment, with regard to the activity of the AroP protein of the present disclosure, a promoter which is one of the gene expression regulatory region on the chromosome encoding the AroP protein may be replaced with a sequence of which activity is strong (e.g., replacement of the wild-type promoter in the expression regulatory region of *aroP* with a pyk or CJ7 promoter), but is not limited thereto.

**In** one embodiment, the microorganism of the present disclosure may produce carnosine.

As used herein, the term "carnosine" refers to a dipeptide composed of beta-alanine and L-histidine.

As used herein, the term "microorganism producing carnosine" refers to a prokaryotic or eukaryotic microorganism capable of producing carnosine within a living organism, and may include both a microorganism prepared by providing the carnosine-producing ability for a parent strain without the carnosine-producing ability, or a microorganism that inherently has the carnosine-producing ability. The carnosine-producing ability may be conferred or enhanced by species improvement.

For example, the microorganism of the present disclosure may be a microorganism having naturally the polypeptide having carnosine synthase activity, the variant polypeptide having carnosine synthase activity, and/or the carnosine-producing ability; or a microorganism in which the variant polypeptide having carnosine synthase activity of the present disclosure, the polynucleotide encoding the same (or the vector including the polynucleotide), the variant of the present disclosure or the polynucleotide encoding the same (or the vector including the polynucleotide) is introduced into and/or the carnosine-producing ability is provided for a parent strain without the polypeptide having carnosine synthase activity of the present disclosure, the variant polypeptide and/or the carnosine-producing ability, but is not limited thereto. Further, the microorganism of the present disclosure may include all of a microorganism including the polypeptide sequence having the carnosine synthase activity of the present disclosure due to mutation of the gene on the chromosome encoding the polypeptide having carnosine synthase activity, a microorganism including the variant polypeptide sequence having carnosine synthase activity of the present disclosure, and a microorganism including the polypeptide having carnosine synthase activity of the present disclosure and/or the variant polypeptide by introducing the vector including the polynucleotide encoding the polypeptide having carnosine synthase activity of the present disclosure and/or the variant polypeptide.

For another example, the microorganism of the present disclosure may be a microorganism having AroP protein of which activity is naturally enhanced; a microorganism having the wild-type AroP; or a microorganism prepared by enhancing AroP in the parent strain without AroP protein, but is not limited thereto. Further, the microorganism of the present disclosure may include all of a microorganism in which AroP is enhanced due to mutation of the regulatory region of the *aroP* gene and a microorganism in which AroP of the present disclosure is enhanced due to introduction of a vector including the *aroP* gene.

As used herein, the term "unmodified microorganism" does not exclude strains including mutation that may occur naturally in microorganisms, and may be a wild-type strain or a natural strain itself or may be a strain before the trait is changed by genetic variation due to natural or artificial factors. For example, the unmodified microorganism may be a strain into which the polypeptide or variant polypeptide having carnosine synthase activity described herein is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

For example, the microorganism having the increased carnosine-producing ability may have about 1% or more, specifically, 30% or more increased carnosine-producing ability, as compared to that of the parent strain before modification or the unmodified microorganism. However, as long as the microorganism has an increased ability of + value, as compared to the production ability of the parent strain before modification or the unmodified microorganism, it is not limited thereto. For another example, the recombinant strain having the increased carnosine-producing ability may have about 1.01 times or more, specifically, about 1.30 times or more increased carnosine-producing ability, as compared to that of the parent strain before modification or the unmodified microorganism, but is not limited thereto. As used herein, the term "about" refers to a range which includes all of ±0.5, ±0.4, ±0.3, ±0.2, ±0.1, etc., and includes all of the values that are equivalent or similar to those following the term "about", but the range is not limited thereto.

For another example, the unmodified microorganism which is a target strain for comparing whether or not the carnosine-producing ability of the present disclosure is increased may be a wild-type *Corynebacterium glutamicum* strain ATCC13032, but is not limited thereto.

In one embodiment, the microorganism including the polypeptide having carnosine synthase activity of the present disclosure, the polynucleotide encoding the same, or the vector including the polynucleotide may have the increased carnosine-producing ability, as compared to a microorganism including any one polypeptide of SEQ ID NOS: 1 and 3 to 7, a polynucleotide encoding the same, or a combination thereof, but is not limited thereto. Polynucleotides of SEQ ID NOS: 10 and 12 to 16 of the present disclosure may encode polypeptides of SEQ ID NOS: 1 and 3 to 7, respectively, and the polypeptides of SEQ ID NOS: 1 and 3 to 7 may be encoded by the polynucleotides of SEQ ID NOS: 10 and 12 to 16, respectively, but are not limited thereto.

In one embodiment, the variant polypeptide of the present disclosure, the polynucleotide encoding the same, or the microorganism including the polynucleotide may have the increased carnosine-producing ability, as compared to a microorganism including any one polypeptide of SEQ ID NOS: 1 to 7, or the polynucleotide encoding the same, or a combination thereof, but is not limited thereto.

In one embodiment, the microorganism in which the activity of AroP protein of the present disclosure is further enhanced may have the increased carnosine-producing ability, as compared to a microorganism in which the activity of AroP protein is not enhanced.

Still another aspect of the present disclosure provides a composition for producing carnosine, the composition including any one or more of the polypeptide having carnosine synthase activity of the present disclosure; the microorganism including any one or more of the polypeptide having carnosine synthase activity of the present disclosure, the polynucleotide encoding the same, and the vector including the polynucleotide; a culture of the microorganism; the variant polypeptide; the microorganism including any one or more of the variant polypeptide, the polynucleotide encoding the same, and the vector including the polynucleotide; and a culture of the microorganism.

The polypeptide having carnosine synthase activity, the variant polypeptide, the polynucleotide, the vector, the microorganism, and the carnosine are as described in other aspects.

The composition of the present disclosure may further include any appropriate excipient that is usually used in the composition for producing carnosine, and examples of the excipient may include a preserving agent, a wetting agent, a dispersing agent, a suspending agent, a buffer, a stabilizing agent, an isotonic agent, etc., but are not limited thereto.

In one specific embodiment, each component present in the composition of the present disclosure may be included in a microbiologically effective amount, or in an amount of being appropriately present in the composition for production.

In the present disclosure, the culture may include any one as long as it includes the culture, such as a dry product, dilution, concentrate, culture filtrate, or fermentation product of the culture.

Still another aspect of the present disclosure provides a method for producing carnosine, the method including the step of culturing the microorganism of the present disclosure in a medium.

The microorganism and the carnosine are as described in other aspects.

As used herein the term "culture" refers to growing the microorganism of the present disclosure in appropriately adjusted environment conditions. The culture procedure of the present disclosure may be performed according to appropriate media or culture conditions known in the art. Such a culture procedure may be easily adjusted according to the selected microorganism by a person skilled in the art. Specifically, the culture may be in a batch type, a continuous type, and/or a fed-batch type, but is not limited thereto.

As used herein, the "medium" refers to a mixture containing, as main ingredients, nutrient materials required for culturing the microorganism of the present disclosure, wherein the medium supplies nutrient materials including water essential for survival and growth, growth factors, etc. Specifically, as for the media and other culture conditions used for culturing the microorganism of the present disclosure, any medium that is used for usual culture of microorganisms may be used without particular limitation. However, the microorganism of the present disclosure may be cultured under aerobic conditions in a common medium containing appropriate carbon sources, nitrogen sources, phosphorus sources, inorganic compounds, amino acids, and/or vitamins, while controlling the temperature, pH, etc. For example, the culture medium for the strains of the genus *Corynebacterium* may be found in a literature ["Manual of Methods for General Bacteriology" by the American Society for Bacteriology (Washington D.C., USA, 1981)].

In the present disclosure, the carbon source may include carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc.; glycerol, propanediol, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of other carbon sources may be variously used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphate sources may include potassium phosphate monobasic, potassium phosphate dibasic, and sodium-containing salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be included. These constituent ingredients or precursors may be added to the medium in a batch or continuous manner. However, the present disclosure is not limited thereto.

Further, the pH of the medium may be adjusted by adding compounds, such as ammonium hydroxide, potassium hydroxide, ammonia, phosphoric acid, and sulfuric acid, to the medium in an appropriate manner during the culture of the microorganism of the present disclosure. In addition, an anti-foaming agent, such as a fatty acid polyglycol ester, may be used to suppress bubble formation during the culture. In addition, oxygen or oxygen-containing gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

In the culture of the present disclosure, the culture temperature may be maintained at 20°C to 45°C, specifically, at 25°C to 40°C, and the culture may be performed for about 10 hours to 160 hours, but is not limited thereto.

The carnosine which is produced by the culture of the present disclosure may be released into the medium or may remain in cells.

In a specific embodiment, the method for producing carnosine of the present disclosure may further include the steps of preparing the microorganism of the present disclosure, preparing a medium for culturing the microorganism, or a combination of these steps (regardless of the order, in any order), for example, before or after the culturing step.

In a specific embodiment, the method for producing carnosine of the present disclosure may further include the step of recovering carnosine from a medium resulting from the culture (a medium in which culture has been performed) or from the microorganism of the present disclosure. The recovering step may be further included after the culturing step.

The recovering may be collecting L-carnosine by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, HPLC, and a combination of these methods may be used, and carnosine may be recovered from the medium or microorganism by using an appropriate method known in the art.

In addition, the method for producing carnosine of the present disclosure may further include a purification step. The purification may be performed by using an appropriate method known in the art. In an exemplary embodiment, when the method of producing carnosine of the present disclosure includes both the recovering step and the purification step, the recovering step and the purification step may be performed continuously or discontinuously regardless of the order, or may be performed simultaneously or integrated into one step, but is not limited thereto.

Still another aspect of the present disclosure provides use of the polypeptide having carnosine synthase activity of the present disclosure; the variant polypeptide; and the microorganism of the present disclosure for producing carnosine.

The polypeptide having carnosine synthase activity, the variant polypeptide, the microorganism, and the carnosine are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Exploration and Selection of Novel Carnosine Synthase

An amino acid sequence of Bacillus-derived carnosine synthase was used as a query sequence, and PSI-BLAST search was performed based on NCBI and Kegg databases. As a result, 20 kinds of candidate genes considered as enzymes capable of synthesizing carnosine and organisms possessing the genes were selected. Among them, seven types of microorganisms and enzymes derived therefrom were selected, as shown in Table 1 below, considering their similarity to the query sequence.

**[Table 1]**

| No. | Microorganism | Protein accession number |
|---|---|---|
| 1 | *Bacillus subtilis 168* | CAB15798.1 |
| 2 | *Streptococcus pneumoniae* | CVN04298.1 |
| 3 | *Alkalihalobacillus halodurans* | MBV7318856.1 |
| 4 | *Priestia megaterium* | WP_116516826.1 |
| 5 | *Pseudomonas syringae* | BAJ15424.1 |
| 6 | *Pseudomonas syringae pv. phaseolicola* | AAZ37741.1 |
| 7 | *Bacillus pumilus* | GM828960.1 |

### Example 2. Construction of Expression Vectors Introduced with Various Carnosine Synthases

Carnosine synthases derived from 7 species (*Bacillus subtilis, Streptococcus pneumoniae, Alkalihalobacillus halodurans, Priestia megaterium, Pseudomonas syringae, Pseudomonas syringae pv. Phaseolicola,* and *Bacillus pumilus*) selected in Example 1 have amino acid sequences of SEQ ID NO: 1 to SEQ ID NO: 7, respectively. Information on genes encoding the above enzymes and the surrounding nucleotide sequences were obtained from the NIH GenBank (accession numbers CAB15798.1, CVN04298.1, MBV7318856.1, WP_116516826.1, BAJ15424.1, AAZ37741.1, and GM828960.1 in that order).

Thereafter, products obtained through gene synthesis based on the obtained sequences were used as templates to perform PCR, thereby obtaining gene fragments for vector construction, respectively.

At this time, Solg^{™} Pfu-X DNA polymerase was used as a polymerase, and PCR amplification conditions consisted of denaturation at 95°C for 3 minutes, 30 cycles of denaturation at 95°C for 20 seconds; annealing at 56°C for 40 seconds; and polymerization at 72°C for 2 minutes, followed by polymerization reaction at 72°C for 5 minutes.

To amplify the gene derived from *Bacillus subtilis,* primers of SEQ ID NOS: 21 and 22 as shown in Table 2 below were prepared, and PCR was performed under the same polymerase and PCR amplification conditions as above, and as a result, 1,419 bp of a gene fragment was obtained.

To amplify the gene derived from *Streptococcus pneumoniae,* primers of SEQ ID NOS: 23 and 24 as shown in Table 2 below were prepared, and PCR was performed in the same manner, and as a result, 1,422 bp of a gene fragment was obtained.

To amplify the gene derived from *Alkalihalobacillus halodurans,* primers of SEQ ID NOS: 25 and 26 as shown in Table 2 below were prepared, and PCR was performed, and as a result, 1,419 bp of a gene fragment was obtained.

To amplify the gene derived from *Priestia megaterium,* primers of SEQ ID NOS: 27 and 28 as shown in Table 2 below were prepared, and PCR was performed in the same manner, and as a result, 1,422 bp of a gene fragment was obtained.

To amplify the gene derived from *Pseudomonas syringae,* primers of SEQ ID NOS: 29 and 30 as shown in Table 2 below were prepared, and PCR was performed in the same manner, and as a result, 1,260 bp of a gene fragment was obtained.

To amplify the gene derived from *Pseudomonas syringae pv. Phaseolicola,* primers of SEQ ID NOS: 31 and 32 as shown in Table 2 below were prepared, and PCR was performed in the same manner, and as a result, 1,245 bp of a gene fragment was obtained.

To amplify the gene derived from *Bacillus pumilus,* primers of SEQ ID NOS: 33 and 34 as shown in Table 2 below were prepared, and PCR was performed in the same manner, and as a result, 1,434 bp of a gene fragment was obtained.

**[Table 2]**

| SEQ ID NO. | Name | Sequence |
|---|---|---|
| 21 | BsLAL_F | GAGGAGATCAAAACAATGGAGCGTAAAACGGTGCTG |
| 22 | BsLAL_R | CGGTGGCGGCCGCTCTAGATCACACCGGGAGGACATATT TC |
| 23 | SpLAL_F | GAGGAGATCAAAACAATGAGCAAGAAGACTGTTCTTG |
| 24 | SpLAL_R | CGCGGTGGCGGCCGCTCTAGATTAGCCCTTCACAAGCTC GT |
| 25 | AhLAL_F | GAGGAGATCAAAACAATGGAACGTAAGACTGTACTGG |
| 26 | AhLAL_R | GCGGTGGCGGCCGCTCTAGATTAGACCGGCAGAACGTAC T |
| 27 | PmLAL_F | GAGGAGATCAAAACAATGAAGCGTAAGACTGTTCTGG |
| 28 | PmLAL_R | CGCGGTGGCGGCCGCTCTAGATTACGTAGTCACAGGCAC GC |
| 29 | PsLAL_F | GAGGAGATCAAAACAATGACGCAGGCAAAAGAGAATA |
| 30 | PsLAL_R | CGCGGTGGCGGCCGCTCTAGATTACTGGACGGAAAAGAG CC |
| 31 | PspLAL_F | GAGATCAAAACAATGAAAAAGATTATTCTCATTGAAACC |
| 32 | PspLAL_R | CGGTGGCGGCCGCTCTAGATTAAAGAGGTTCTGACGAAT CG |
| 33 | BpLAL_F | GAGGAGATCAAAACAATGGTCCTTAGCCTGTCGAAAAAG |
| 34 | BpLAL_R | CGGTGGCGGCCGCTCTAGATCAACCTTTCACCAACTCATC CATAAG |
| 35 | Po2_F | CCTGCAGCCCGGGGGATCCCAATAATCGTGAATTTTGGCA GC |
| 36 | Po2_R | GGCGGCCGCTCTAGATGTTTTGATCTCCTCCAATAATCTAT G |

Further, in order to obtain a promoter, o2 promoter (US 10273491 B2) was used as a template and primers of SEQ ID NOS: 35 and 36 as shown in Table 2 above were prepared to perform PCR.

The amplified o2 promoter region, the gene fragments derived from 7 species of microorganisms, and pCES208 vector digested with Xbal restriction enzyme ("Construction of heat-inducible expression vector of Corynebacterium glutamicum and C. ammoniagenes: fusion of lambda operator with promoters isolated from C. ammoniagenes." Journal of microbiology and biotechnology 18.4 (2008): 639-647.) were ligated using an In-fusion cloning kit to obtain each gene expression vector.

Thus, the vector containing the *Bacillus subtilis-*derived gene, the vector containing the *Streptococcus pneumoniae-*derived gene, the vector containing the *Alkalihalobacillus halodurans-*derived gene, the vector containing the *Priestia megaterium*-derived gene, the vector containing the *Pseudomonas* syringae-derived gene, the vector containing the *Pseudomonas syringae pv. Phaseolicola-*derived gene, and the vector containing the *Bacillus pumilus-*derived gene were named "pCES208-Po2-BsLAL", "pCES208-Po2-SpLAL", "pCES208-Po2-AhLAL", "pCES208-Po2-PmLAL", "pCES208-Po2-PsLAL", "pCES208-Po2-PspLAL", and "pCES208-Po2-BpLAL", respectively.

### Example 3. Preparation of Strain of genus Corynebacterium Producing Beta-alanine and L-histidine

To evaluate the carnosine synthesis activity of the above enzymes, it was attempted to prepare a strain of the genus *Corynebacterium* producing beta-alanine and L-histidine. Accordingly, a vector was prepared to insert *panD* into the strain's genomic DNA, the gene required for converting aspartic acid produced in the *Corynebacterium glutamicum* strain into beta-alanine.

To amplify the *panD* gene, the chromosomal DNA of wild-type *Corynebacterium glutamicum* ATCC13032 was used as a template, and primers of SEQ ID NO: 37 and SEQ ID NO: 38 as shown in Table 3 below were prepared, and PCR was performed in the same manner as in Example 2. As a result, 411 bp of a gene fragment was obtained.

To obtain the o2 promoter derived from *Corynebacterium glutamicum,* PCR was performed using genomic DNA of *Corynebacterium glutamicum* as a template and primers of SEQ ID NOS: 39 and 40 as shown in Table 3 below.

**[Table 3]**

| SEQ ID NO. | Name | Sequence |
|---|---|---|
| 37 | panD_F | GGAGGAGATCAAAACAATGCTGCGCACCATCCTCG |
| 38 | panD_R | CGTAGCCTTGGAATCCACTAAATGCTTCTCGACGTC AAAAG |
| 39 | Po2panD_F | GGCTCCCGCGGTGAACAATAATCGTGAATTTTGGCA GC |
| 40 | Po2panD_R | GGATGGTGCGCAGCATTGTTTTGATCTCCTCCAATAA TC |
| 41 | check_Po2-panD_F | CCTGGGCTAGCGGTGTAG |
| 42 | check_Po2-panD_R | CTGCTCTTAAATCCGCGG |

The amplified o2 promoter region, the *panD* gene fragment, and a pDZTn vector for chromosome transformation (Korean Patent No. 10-1126041) which had been digested with Spel restriction enzyme were cloned using the Gibson assembly method (DG Gibson et al., NATURE METHODS, VOL.6 NO.5, MAY 2009, NEBuilder HiFi DNA Assembly Master Mix) to obtain a recombinant plasmid, and the obtained plasmid was named pDZTn-Po2-panD. Cloning was performed by mixing the Gibson assembly reagent and each gene fragment in the calculated molar quantity and then storing the mixture at 50°C for 1 hour.

The prepared pDZTn-Po2-panD vector was transformed into the wild-type *Corynebacterium glutamicum* ATCC13032 strain by electroporation (Appl. Microbiol.Biotechnol. (1999) 52:541-545), and then through a second crossover process, a strain was obtained in which one copy of the Po2-panD gene was inserted between the transposon genes on the chromosome. Genetic manipulation was confirmed through PCR and genome sequencing using primers of SEQ ID NO: 41 and 42 as shown in Table 3, which are able to amplify the external regions of the homologous recombination upstream and downstream regions where the gene was inserted.

The strain thus obtained was named *Corynebacterium glutamicum* "ATCC13032::Po2-panD".

### Example 4. Preparation of Strains of genus Corynebacterium Introduced with Various Carnosine Synthases

The vectors prepared in Example 2 were transformed into the *Corynebacterium glutamicum* ATCC13032::Po2-panD strain prepared in Example 3 by electroporation (Appl. Microbiol.Biotechnol. (1999) 52:541-545), and thus 7 kinds of strains into which each gene was introduced were obtained, respectively.

At this time, the pCES208-Po2-BsLAL-introduced strain, the pCES208-Po2-SpLAL-introduced strain, the pCES208-Po2-AhLAL-introduced strain, the pCES208-Po2-PmLAL-introduced strain, the pCES208-Po2-PsLAL-introduced strain, the pCES208-Po2-PspLAL-introduced strain, and the pCES208-Po2-BpLAL-introduced strain were named "ATCC13032::Po2-panD-BsLAL", "ATCC13032:: Po2-panD-SpLAL", "ATCC13032:: Po2-panD-AhLAL", "ATCC13032:: Po2-panD-PmLAL", "ATCC13032:: Po2-panD-PsLAL", "ATCC13032:: Po2-panD-PspLAL", and "ATCC13032:: Po2-panD-BpLAL", respectively.

### Example 5. Verification of Carnosine Production in Strains of genus Corynebacterium Introduced with Various Carnosine Synthases

A fermentation titration was conducted to verify carnosine production of 7 kinds of *Corynebacterium glutamicum* strains prepared in Example 4. Colonies of each strain were subcultured on a nutrient medium and then cultured on a fermentation medium for 48 hours. The medium compositions used here are as follows.

### <Nutrient medium>

1% glucose, 0.5% beef extract, 1% polypeptone, 0.25% sodium chloride, 0.5% yeast extract, 2% agar, 0.2% urea, pH 7.2

### <Fermentation medium>

6% glucose, 3% calcium carbonate, 2% ammonium sulfate, 1% sugarcane molasses, 0.1% yeast extract, 0.4% monopotassium phosphate, 0.3% magnesium sulfate, 0.026% isoleucine, 36 mg/L nicotinamide, 2.5 mg/L iron sulfate, 1.175 mg/L manganese sulfate, 0.05 mg/L biotin

After the culture was completed under the above conditions, the carnosine concentration in each medium was measured using HPLC. The carnosine concentrations produced by the 7 kinds of strains are shown in Table 4 below.

**[Table 4]**

| Name of strain | Carnosine (g/L) |
|---|---|
| ATCC13032::Po2-panD-BsLAL | 0.9 |
| ATCC13032::Po2-panD-SpLAL | 1.5 |
| ATCC13032::Po2-panD-AhLAL | 0.9 |
| ATCC13032::Po2-panD-PmLAL | 0.3 |
| ATCC13032::Po2-panD-PsLAL | 0 |
| ATCC13032::Po2-panD-PspLAL | 0 |
| ATCC13032::Po2-panD-BpLAL | 1.1 |

As shown in Table 4, ATCC13032::Po2-panD-SpLAL, into which the enzyme derived from *Streptococcus pneumoniae* among various carnosine synthases was introduced, produced 1.5 g/L of carnosine, indicating that this is about 0.6 g/L (about 67%) higher than the carnosine concentration produced by ATCC13032::Po2-panD-BsLAL, into which the enzyme derived from *Bacillus subtilis* was introduced, previously reported, and is about 0.4 g/L (about 36%) higher than the carnosine concentration produced by ATCC13032::Po2-panD-BpLAL, into which the enzyme derived from *Bacillus pumilus* was introduced.

The above results confirmed that the enzyme derived from *Streptococcus pneumoniae* had the highest carnosine synthesis activity, and in some cases, carnosine was not produced depending on the origin of carnosine synthase.

### Example 6. Carnosine Production by Corynebacterium Strains introduced with Variant Streptococcus pneumoniae-derived Enzymes

### Example 6-1. Preparation of Strains introduced with Variant Streptococcus pneumoniae-derived Enzymes

Two kinds of amino acid variations were introduced into the *Streptococcus pneumoniae-*derived enzyme with the highest carnosine synthesis activity, selected through Example 5. In detail, the improvement in carnosine synthesis activity was verified by substituting glutamic acid and lysine for asparagine and histidine which are amino acids at positions 108 and 378 in the enzyme sequence, respectively.

For this purpose, first, in order to substitute glutamic acid for asparagine which is the amino acid at position 108 of SEQ ID NO: 2, a set of primers (mutagenic primers) of SEQ ID NOS: 43 and 44 as shown in Table 5 below were prepared using pCES208-Po2-SpLAL prepared in Example 2 as a template.

**[Table 5]**

| Variant SpLAL plasmid | SEQ ID NO. # | Nucleotide sequence |
|---|---|---|
| pCES208-Po2-SpLAL N108E | 43 | CGATTACGAC**GAA**CGAAGAACTGTTTATTGCCCCAA |
| | 44 | TTGGGGCAATAAACAG**TTC**TTCGTTCGTCGTAATCG |
| pCES208-Po2-SpLAL N108E/H378K | 45 | CCACACGTTTTATGTGGCAGATTGTAAACTTTATCCTCAACATTTTAA AGAAT |
| | 46 | ATTCTTTAAAATGTTGAGGATAAAGTTTACAATCTGCCACATAAAACG TGTGG |

A PCR mixture as shown in Table 6 below was prepared using the above primer set, and site-direction mutagenesis PCR was performed using the cycle shown in Table 7 below.

**[Table 6]**

| Site-Direction Mutagenesis PCR composition | Unit (ul) |
|---|---|
| 10X pfu-X Buffer | 5 |
| 10 mM dNTP Mix | 1 |
| pfu-X Polymerase | 1 |
| Mutagenic forward primer (5 pmol) | 2 |
| Mutagenic reverse primer (5 pmol) | 2 |
| Template DNA, 200 ng/ul | 1 |
| dH₂O | 38 |
| Total | 50 |

**[Table 7]**

| Cycle | Temperature | Time |
|---|---|---|
| 1 | 95°C | 1 min |
| 18 | 95°C | 50 sec |
| | 60°C | 50 sec |
| | 68°C | 9 min |
| 1 | 68°C | 7 min |

1 µl of DpnI restriction enzyme was added to the mixture where PCR was completed and left at 37°C for 1 hour. 3 µl of DpnI-treated DNA was transformed into DH5a competent cells to obtain a pCES208-Po2-SpLAL N108E plasmid.

Additionally, in order to substitute lysine for histidine which is the amino acid at position 378, a pCES208-Po2-SpLAL N108E/H378 plasmid was obtained in the same manner as above using pCES208-Po2-SpLAL N108E plasmid prepared above as a template and a set of primers (mutagenic primers) of SEQ ID NOS: 45 and 46 as shown in Table 5. Through sequencing, substitution with each mutation as indicated in the plasmid of Table 5 was confirmed, and as a result, the sequence of SEQ ID NO: 8 was obtained.

The pCES208-Po2_SpLAL N108E/H378K vector constructed above was transformed into the *Corynebacterium glutamicum* ATCC13032::Po2-panD strain prepared in Example 3 by electroporation, and then a strain into which the variant SpLAL gene (N108E/H378K) was inserted was obtained. The strain was named "ATCC13032::Po2-panD-SpLAL N108E/H378K".

### Example 6-2: Verification of carnosine production in Corynebacterium strains introduced with variant Streptococcus pneumoniae-derived enzymes

In order to examine the carnosine production of the *Corynebacterium glutamicum* ATCC13032::Po2-panD-SpLAL N108E/H378K strain prepared in Example 6-1, carnosine production was measured using HPLC after culturing in the same manner as in Example 5.

**[Table 8]**

| Name of strain | Carnosine production (g/L) |
|---|---|
| ATCC13032::Po2-panD-SpLAL | 1.5 |
| ATCC13032::Po2-panD-SpLAL N108E/H378K | 2.1 |

As shown in Table 8, it was confirmed that the concentration of carnosine produced by the microorganism into which the variant SpLAL enzyme (N108E/H378K) was introduced was about 0.6 g/L (about 40%) higher than the concentration of carnosine produced by the microorganism into which the wild-type SpLAL enzyme was introduced.

The above results indicate that the N108E/H378K variation increases the carnosine production ability of the microorganism having the enzyme.

### Example 7. Improvement of Carnosine Productivity of AroP-enhanced Corynebacterium Strain

### Example 7-1. Preparation of AroP-enhanced strain

To enhance AroP of the *Corynebacterium* strain, a vector for introducing pyk promoter and cj7 promoter (US 7662943 B2) was constructed.

To amplify the pyk promoter, primers of SEQ ID NOS: 47 and 48 as shown in Table 9 below were prepared using the chromosomal DNA of the wild-type *Corynebacterium glutamicum* ATCC13032 as a template, and PCR was performed in the same manner as in Example 2, and as a result, 500 bp of a gene fragment was obtained. To obtain gene fragments required for insertion into the host genomic DNA, SEQ ID NOS: 49 and 50 and SEQ ID NOS: 51 and 52 as shown in Table 9 were prepared, and PCR was performed in the same manner as in Example 2 to obtain 501 bp and 500 bp of gene fragments.

**[Table 9]**

| SEQ ID NO. | Name | Sequence |
|---|---|---|
| 47 | pyk_F | GGTTTACGGTTGATACGTATTTGCACTCTACGTAGCTG G |
| 48 | pyk_R | CTTCATTAGATTTAGCCATGCCCATAAGCCTAGTACGTC |
| 49 | Aarm_F | gagctcggtacccggggatccGTTCCCCCTGGAGTCCG |
| 50 | Aarm_pyk_R | CTACGTAGAGTGCAAATACGTATCAACCGTAAACCCAC ATG |
| 51 | Barm_pyk_F | GTACTAGGCTTATGGGCATGGCTAAATCTAATGAAGGG C |
| 52 | Barm_R | cctgcaggtcgactctagaCCAATGATGAGGAAAGCGATG |

The amplified pyk promoter region and the two gene fragments obtained above were ligated to a pDZ vector (Korean Patent No. 10-0924065 and International Patent Publication No. 2008-033001), which had been digested with restriction enzymes BamHI and Xbal, using an In-fusion cloning kit to prepare a vector for gene introduction, which was named "pDZ-pyk(AroP)".

In addition, in order to amplify the CJ7 promoter, primers of SEQ ID NOS: 53 and 54 as shown in Table 10 below were prepared using the chromosomal DNA of the wild-type *Corynebacterium glutamicum* ATCC13032 as a template, and PCR was performed in the same manner as in Example 2, and as a result, 317 bp of a gene fragment was obtained. To obtain gene fragments required for insertion into the host genomic DNA, SEQ ID NOS: 49 and 55 and SEQ ID NOS: 56 and 52 as shown in Tables 9 and 10 were prepared, and PCR was performed in the same manner as in Example 2 to obtain 501 bp and 500 bp of gene fragments.

**[Table 10]**

| SEQ ID NO. | Name | Sequence |
|---|---|---|
| 53 | cj7_F | GGGTTTACGGTTGATACAGAAACATCCCAGCGCTACTAAT AG |
| 54 | cj7_R | CTTCATTAGATTTAGCCATAGTGTTTCCTTTCGTTGGGTAC |
| 55 | Aarm_cj7_R | GTAGCGCTGGGATGTTTCTGTATCAACCGTAAACCCACAT G |
| 56 | Barm_cj7_F | CCCAACGAAAGGAAACACTATGGCTAAATCTAATGAAGGG C |

The amplified CJ7 promoter region and the two gene fragments obtained above were ligated to the pDZ vector (Korean Patent No. 10-0924065 and International Patent Publication No. 2008-033001), which had been digested with restriction enzymes BamHI and Xbal, using an In-fusion cloning kit to prepare a vector for gene introduction, which was named "pDZ-cj7(AroP)".

The prepared pDZ-pyk(AroP) and pDZ-cj7(AroP) vectors were transformed into the ATCC13032::Po2-panD-SpLAL N108E/H378K strain prepared in Example 6-1 by electroporation (Appl. Microbiol.Biotechnol. (1999) 52:541-545), respectively, and then through a second crossover process, a strain was obtained in which the promoter was inserted before the AroP gene on the chromosome. The corresponding genetic manipulation was confirmed through PCR and genome sequencing using primers of SEQ ID NOS: 57 (check_AroP_F; CTCTGCGGTCCCGCGGAC) and 58 (check_AroP_R; CGTGATCACCGATGAAGTTTG), which are able to amplify the external regions of the homologous recombination upstream and downstream regions where the corresponding gene was inserted, respectively.

The *Corynebacterium glutamicum* strains thus obtained were named "ATCC13032::Po2-panD-SpLAL (N108E/H378K)-PpykAroP" and "ATCC13032::Po2-panD-SpLAL(N108E/H378K)-Pcj7AroP", respectively.

### Example 7-2. Verification of Carnosine Production of AroP-enhanced strains

To examine the carnosine production of ATCC13032::Po2-panD-SpLAL (N108E/H378K)-PpykAroP strain and ATCC13032::Po2-panD-SpLAL(N1 08E/H378K)-Pcj7 AroP strain, each strain was cultured in the same manner as in Example 5, and then the production of carnosine was measured using HPLC.

**[Table 11]**

| Name of strain | Carnosine production (g/L) |
|---|---|
| ATCC13032::Po2-panD-SpLAL N108E/H378K | 2.1 |
| ATCC13032::Po2-panD-SpLAL (N108E/H378K)-PpykAroP | 2.5 |
| ATCC13032::Po2-panD-SpLAL(N108E/H378K)-Pcj7AroP | 2.7 |

As shown in Table 11, it was confirmed that the concentration of carnosine produced by the pyk promoter-introduced ATCC13032::Po2-panD-SpLAL (N108E/H378K)-PpykAroP was about 0.4 g/L (about 19%) higher than the concentration of carnosine produced by ATCC13032::Po2-panD-SpLAL N108E/H378K, and the concentration of carnosine produced by the cj7 promoter-introduced ATCC13032::Po2-panD-SpLAL(N108E/H378K)-Pcj7AroP was about 0.6 g/L (about 29%) higher than the concentration of carnosine produced by ATCC13032::Po2-panD-SpLAL N108E/H378K.

The above results indicate that the AroP enhancement increases the carnosine production ability of the microorganism.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A polypeptide having carnosine synthase activity, the polypeptide comprising an amino acid sequence of SEQ ID NO: 2.

2. A polynucleotide encoding the polypeptide having carnosine synthase activity of claim 1.

3. A microorganism comprising any one or more of the polypeptide having carnosine synthase activity of claim 1, a polynucleotide encoding the same, and a vector comprising the polynucleotide.

4. The microorganism of claim 3, wherein the microorganism produces carnosine.

5. The microorganism of claim 3, wherein an activity of Aromatic amino acid transport protein (AroP) protein is further enhanced.

6. The microorganism of claim 5, wherein the AroP protein comprises an amino acid sequence of SEQ ID NO: 19.

7. The microorganism of claim 3, wherein the microorganism is a microorganism of the genus *Corynebacterium.*

8. The microorganism of claim 3, wherein the microorganism is *Corynebacterium glutamicum.*

9. A variant polypeptide having carnosine synthase activity, wherein an amino acid corresponding to position 108 and an amino acid corresponding to position 378 from the N-terminus of SEQ ID NO: 2 are substituted with different amino acids.

10. The variant polypeptide of claim 9, wherein the amino acid corresponding to position 108 and the amino acid corresponding to position 378 from the N-terminus of SEQ ID NO: 2 are substituted with glutamic acid and lysine, respectively.

11. The variant polypeptide of claim 9, wherein asparagine and histidine which are respectively the amino acids corresponding to positions 108 and 378 from the N-terminus of SEQ ID NO: 2 are substituted with glutamic acid and lysine, respectively.

12. The variant polypeptide of claim 9, comprising an amino acid sequence of SEQ ID NO: 8.

13. A polynucleotide encoding the variant polypeptide of claim 9.

14. A microorganism comprising any one or more of the variant polypeptide of claim 9, a polynucleotide encoding the same, and a vector comprising the polynucleotide.

15. The microorganism of claim 14, wherein the microorganism produces carnosine.

16. The microorganism of claim 14, wherein an activity of Aromatic amino acid transport protein (AroP) protein is further enhanced.

17. A composition for producing carnosine, the composition comprising any one or more of the polypeptide of claim having carnosine synthase activity 1; a microorganism comprising any one or more of the polypeptide having carnosine synthase activity of claim 1, a polynucleotide encoding the same, and a vector comprising the polynucleotide; a culture of the microorganism; the variant polypeptide of any one of claims 9 to 12; a microorganism comprising any one or more of the variant polypeptide of any one of claims 9 to 12, a polynucleotide encoding the same, and a vector comprising the polynucleotide; a culture of the microorganism.

18. The composition of claim 17, wherein the microorganism has the further enhanced activity of Aromatic amino acid transport protein (AroP) protein.

19. A method for producing carnosine, the method comprising the step of culturing, in a medium, the microorganism of any one of claims 3 to 8, and 14 to 16.

20. The method of claim 19, further comprising the step of recovering carnosine from any or more of the microorganism, the culture thereof, and the medium.

21. Use of the microorganism of any one of claims 3 to 8, and 14 to 16 for producing carnosine.
